# EUROPEAN PATENT APPLICATION

(11) **EP 4 834 687 A1**
(43) Date of publication of application: **04.11.2026**
(21) Application number: 25218730.7
(22) Date of filing: 26.11.2025
(51) Int. Cl.: B60W 30/10, B60W 30/16, B60W 40/08, B60W 50/00, B60W 60/00, B60W 50/14, B60W 10/04, B60W 10/18, B60W 10/20

(54) **VEHICLE AND METHOD FOR CONTROLLING THE SAME**

(30) Priority: 28.04.2025 KR 20250055411
(71) Applicant: Hyundai Motor Company, Seoul 06797 (KR); Kia Corporation, Seoul 06797 (KR)
(72) Inventor: KIM, Dong Sun, 18280 Hwaseong-si, Gyeonggi-do (KR); CHOI, Eun Young, 18280 Hwaseong-si, Gyeonggi-do (KR); KIM, Su Hyun, 18280 Hwaseong-si, Gyeonggi-do (KR); KIM, Jong Chul, 18280 Hwaseong-si, Gyeonggi-do (KR); LEE, Jin Ug, 18280 Hwaseong-si, Gyeonggi-do (KR)
(74) Representative: Hoffmann Eitle

(57) **Abstract**

A vehicle and a method for controlling same are disclosed. An autonomous vehicle control device according to an example of the present invention includes: a memory; and a processor electrically or adaptively connected to the memory, wherein the processor may be configured to: determine a driver's state when a hands-off state of the driver is detected through a sensor unit of an autonomous vehicle, and control at least one of a driving operation or a warning output of the autonomous vehicle based on the determined driver's state.

## Description

### CROSS-REFERENCE TO RELATED APPLICATION

This application claims priority to Korean Patent Application No. 10-2025-0055411, filed on April 28, 2025, the disclosure of which is incorporated herein by reference in its entirety.

### FIELD OF TECHNOLOGY

Various examples of the present invention relate to a technique for operating a driving assistance function more efficiently in autonomous driving.

### BACKGROUND

Advances in autonomous driving enable vehicles to automatically adjust inter-vehicle distance maintenance, collision avoidance, and lane keeping functions.

In certain autonomous driving sections, a hands-free state is possible in which a driver drives with his or her hands off a steering wheel.

However, the existing system does not sufficiently reflect the driver's state, and the risk of an accident may increase in a hands-off state.

### SUMMARY

The technical problem to be achieved by the present invention has been devised to solve the above-described problem, and an object of the present invention is to automatically adjust the driving assistance sensitivity based on the state of the driver.

The problems to be solved by the present invention are not limited to the problems mentioned above, and other problems not mentioned will be clearly understood by a person skilled in the art from the following description.

An autonomous vehicle control device according to an example of the present disclosure may comprise: a memory; and a processor electrically or adaptively coupled to the memory, wherein the processor may be configured to: determine a driver's state when a hands-off state of the driver is detected through a sensor unit of an autonomous vehicle; and/or control at least one of a driving operation or a warning output of the autonomous vehicle based on the determined driver's state.

In the autonomous vehicle control device according to some examples of the present disclosure, the processor may be configured to control at least one of a driving operation or a warning output of the autonomous vehicle depending on whether the hands-off of the driver in the autonomous vehicle is a permitted state or a non-permitted state.

In the autonomous vehicle control device according to some examples of the present disclosure, the driver's state may comprise at least one of a contact area of a user's body on a steering wheel of the autonomous vehicle, a position of the user's body, or an object adjacent to the user's body.

In the autonomous vehicle control device according to some examples of the present disclosure, the driver's state may comprise a gaze direction of the driver.

In the autonomous vehicle control device according to some examples of the present disclosure, the driver's state may comprise at least one of an eye closing ratio of the driver, an eye closing duration of the driver, or a combination thereof.

In the autonomous vehicle control device according to some examples of the present disclosure, the driving operation of the autonomous vehicle may comprise control of an inter-vehicle distance of the autonomous vehicle.

In the autonomous vehicle control device according to some examples of the present disclosure, the processor may be configured to control an inter-vehicle distance of the autonomous vehicle to be increased more than a preset inter-vehicle distance when a gaze of the driver is out of a driving direction for more than a preset time or an eye-closed state is confirmed.

In the autonomous vehicle control device according to some examples of the present disclosure, the driving operation of the autonomous vehicle may comprise control of at least one of a steering angle or a steering torque of the autonomous vehicle.

In the autonomous vehicle control device according to some examples of the present disclosure, the control of the warning output may comprise control of at least one of an intensity of the warning, a duration of the warning, and/or a repetition period of the warning.

In the autonomous vehicle control device according to some examples of the present disclosure, the processor may be configured to output, via a display, a control state associated with at least one of the driving operation or the warning output of the autonomous vehicle.

A method for controlling an autonomous vehicle according to an example of the present disclosure may comprise: determining a driver's state when a hands-off state of the driver is detected through a sensor unit of an autonomous vehicle; and/or controlling at least one of a driving operation or a warning output of the autonomous vehicle based on the determined driver's state.

In the method for controlling an autonomous vehicle according to some examples of the present disclosure, the controlling may be performed depending on whether the hands-off of the driver in the autonomous vehicle is a permitted state or a non-permitted state.

In the method for controlling an autonomous vehicle according to some examples of the present disclosure, the driver's state may comprise at least one of a contact area of a user's body on a steering wheel of the autonomous vehicle, a position of the user's body, and/or an object adjacent to the user's body.

In the method for controlling an autonomous vehicle according to some examples of the present disclosure, the driver's state may comprise a gaze direction of the driver.

In the method for controlling an autonomous vehicle according to some examples of the present disclosure, the driver's state may comprise at least one of an eye closing ratio of the driver, an eye closing duration of the driver, and/or a combination thereof.

In the method for controlling an autonomous vehicle according to some examples of the present disclosure, the driving operation of the autonomous vehicle may comprise control of an inter-vehicle distance of the autonomous vehicle.

In the method for controlling an autonomous vehicle according to some examples of the present disclosure, the controlling the driving operation may comprise controlling the inter-vehicle distance of the autonomous vehicle to be increased more than a preset inter-vehicle distance when a gaze of the driver is out of a driving direction for more than a preset time or an eye-closed state is confirmed.

In the method for controlling an autonomous vehicle according to some examples of the present disclosure, the driving operation of the autonomous vehicle may comprise control of at least one of a steering angle or a steering torque of the autonomous vehicle.

In the method for controlling an autonomous vehicle according to some examples of the present disclosure, the controlling the warning output may comprise control of at least one of an intensity of the warning, a duration of the warning, or a repetition period of the warning.

The method for controlling an autonomous vehicle according to some examples of the present disclosure further may comprise outputting, via a display, a control state associated with at least one of the driving operation or the warning output of the autonomous vehicle.

According to an example of the present disclosure, by automatically adjusting the driving assistance sensitivity based on the state of a driver, the system may actively intervene and improve safety even when the driver is inadvertent.

For instance, aspects described herein may relate to a computing device for monitoring a driver during autonomous operation of a vehicle, the computing device comprising one or more processors and memory storing instructions that, when executed by the one or more processors, cause the computing device to perform various steps. The computing device may generate a behavior pattern of a driver of the vehicle by collecting, via one or more sensors of the vehicle, first sensor data corresponding to a body of the driver. The computing device may receive, from the one or more sensors of the vehicle and during autonomous driving operations of the vehicle, second sensor data corresponding to the body of the driver. The computing deivce may identify a degree of driver attentiveness by comparing the second sensor data to the behavior pattern of the driver. The computing device may cause, based on determining that the degree of driver attentiveness satisfies a threshold, the vehicle to operate in a safe driving mode by increasing an inter-vehicle distance of the vehicle to a value greater than a preset inter-vehicle distance.

The effects of the present invention are not limited to the effects mentioned above, and other effects not mentioned will be clearly understood by a person skilled in the art from the following description.

### BRIEF DESCRIPTION OF THE DRAWINGS

The above and other objects, features and advantages of the present disclosure will become more apparent to those of ordinary skill in the art by describing one or more example examples thereof in detail with reference to the accompanying drawings, in which:
FIG. 1 is a configuration diagram of a vehicle.
FIG. 2 is a flowchart of an operation of controlling a vehicle.
FIG. 3 is a specific flowchart of an operation of controlling a vehicle.
FIGS. 4 to 5 are exemplary diagrams of sensitivity setting tables.

### DETAILED DESCRIPTION

Hereinafter, one or more example examples of the present disclosure will be described in detail with reference to the accompanying drawings.

However, the technical idea of the present disclosure is not limited to the example examples as described herein but may be implemented in various different forms, and within the scope of the technical idea of the present disclosure, one or more among components in the example examples may be used by being selectively combined and substituted.

Further, unless specifically defined and described, terms used in the example examples of the present disclosure (including technical and scientific terms) may be interpreted as meanings which are generally understood by those skilled in the art to which the present disclosure pertains, and commonly used terms such as terms defined in the dictionary may be interpreted in consideration of the contextual meaning of the related art.

The terms used in the example examples of the present disclosure are for the purpose of describing the example examples only and are not intended to limit the disclosure.

In the present specification, the singular forms may include the plural forms unless the context clearly dictates otherwise. For purposes of this application and the claims, using the exemplary phrase "at least one of: A; B; or C" or "at least one of A, B, or C," the phrase means "at least one A, or at least one B, or at least one C, or any combination of at least one A, at least one B, and at least one C. Further, exemplary phrases, such as "A, B, or C", "at least one of A, B, and C", "at least one of A, B, or C", etc. as used herein may mean each listed item or all possible combinations of the listed items. For example, "at least one of A or B" may refer to (1) at least one A; (2) at least one B; or (3) at least one A and at least one B.

In addition, in describing a component of example examples of the present disclosure, terms such as first, second, A, B, (a), (b), etc., may be used.

These terms are only for distinguishing the component from other components, and the essence, sequence, or order of the component is not limited by the terms.

In addition, when a component is described as being "linked," "coupled," or "connected" to another component, the component is not only directly linked, coupled, or connected to another component, but also "linked," "coupled," or "connected" to another component with still another component disposed between the component and the other component.

Further, when a component is described as being formed or disposed "on (above) or under (below)" of another component, the term "on (above) or under (below)" includes not only when two components are in direct contact with each other, but also when one or more of other components are formed or disposed between the two components. Further, when a component is described as being "on (above) or below (under)," the description may include the meanings of an upward direction and a downward direction based on one component.

The term "module" or "unit" used in the specification means a software and/or hardware component, and the "module" or "unit" performs certain operations/functions/roles. However, the "module" or "unit" is not construed as being limited to software or hardware. The "module" or "unit" may be configured to be in an addressable storage medium or to execute one or more processors. Therefore, as an example, the "module" or "unit" may include at least one of components such as software components, object-oriented software components, class components, and task components, processes, functions, attributes, procedures, sub-routines, segments of program codes, drivers, firmware, micro-codes, circuits, data, databases, data structures, tables, arrays, or variables. Functions provided in the components, "modules", or "units" may be combined into a smaller number of components, "modules", or "units" or further divided into additional components, "modules", or "units".

In the present disclosure, the "module" or "unit" may be realized as a processor and a memory. The "processor" should be widely construed to include a general-purpose processor, a central processing unit (CPU), a microprocessor, a digital signal processor (DSP), a microcontroller, a state machine, or the like. In some environments, the "processor" may refer to an application-specific integrated circuit (ASIC), a programmable logic device (PLD), or a field-programmable gate array (FPGA), and the like. For example, the "processor" may refer to a combination of processing devices such as a combination of a DSP and a microprocessor, a combination of a plurality of microprocessors, a combination of one or more microprocessors combined with a DSP core, or any other such combination. Moreover, the "memory" should be widely construed to include any electronic component capable of storing electronic information. The "memory" may refer to various types of processor-readable medium such as a random-access memory (RAM), a read only memory (ROM), a non-volatile random access memory (NVRAM), a programmable read only memory (PROM), an erasable programmable read only memory (EPROM), an electrically erasable programmable read only memory (EEPROM), a flash memory, a magnetic or optical data storage device, and registers. When the processor can read information from a memory and/or record the information in the memory, the memory may be in a state of electronic communication with a processor. Memory integrated into a processor is in a state of electronic communication with the processor.

The one or more features described herein may be provided as a computer program stored in a computer-readable recording medium in order to be executed on a computer. The medium may either continuously store a computer-executable program or temporarily store the program for execution or download. Furthermore, the medium may be a variety of recording or storage means in the form of a single hardware device or multiple combined hardware devices, and is not limited to media directly connected to some computer system but may also be distributed across a network. Examples of such media include magnetic media such as a hard disk, a floppy disk, or a magnetic tape, optical recording media such as a CD-ROM or a DVD, magneto-optical media such as a floptical disk, and a ROM, RAM, or flash memory, among others, configured to store program instructions. Additional examples of such media include media or storage media that are managed by an app store that distributes applications or by various other sites or servers that provide or distribute software.

In a hardware implementation, processing units used for performing the techniques may be implemented within one or more ASICs, DSPs, digital signal processing devices, programmable logic devices, field-programmable gate arrays, processors, controllers, microcontrollers, microprocessors, electronic devices, or computers or combinations thereof designed to perform the functions described in the present disclosure.

In the various flowcharts of the present disclosure, at least some of the steps may be omitted or the mutual order thereof may be changed, and at least some of the various examples of the present document may be performed at a specific point in time of each step of the flowcharts. The operations of the various flowcharts of the present document may be performed by at least one of the control device 100, the processor 130, or the autonomous vehicle 10. Furthermore, redundant content in the drawings of the present document may be omitted. In the present document, 'the control device 100' may also be referred to as 'the autonomous vehicle control device 100'.

The term "autonomous driving system" or "driver control assistance system" as used in this document refers to hardware and software capable of assisting the driver in continuously controlling the longitudinal and lateral movements of the vehicle. This driver control assistance system may be referred to as the "system" in this document.

The term "feature" as used in this document may refer to a specific function of the system that provides assistance to the driver under defined traffic scenarios, conditions, and system boundaries.

The term "dynamic control" as used in this document may refer to the real-time performance of operational and tactical functions required to move the vehicle. This may include control of the vehicle's lateral and longitudinal movements, monitoring of the road environment, response to events in the road traffic environment, operational planning, and signal delivery.

The term "system boundaries" as used in this document may refer to verifiable or measurable limits or conditions set by the manufacturer, up to or within the range of the conditions that affect the ability of the system or its features to operate as intended and to provide assistance to the driver.

The term 'Hands off' as used in this document refers to a state where the driver is not holding the steering wheel, which can occur regardless of whether it is permitted by the system.

The term 'Hands free' as used in this document can be interpreted as a feature that allows the vehicle to operate without the driver's steering input under specific conditions. Therefore, 'Hands free' can mean a state where 'Hands off' is permitted by the system in certain segments.

An automation level of an autonomous driving vehicle may be classified as follows, according to the American Society of Automotive Engineers (SAE). At autonomous driving level 0, the SAE classification standard may correspond to "no automation," in which an autonomous driving system is temporarily involved in emergency situations (e.g., automatic emergency braking) and/or provides warnings only (e.g., blind spot warning, lane departure warning, etc.), and a driver is expected to operate the vehicle. At autonomous driving level 1, the SAE classification standard may correspond to "driver assistance," in which the system performs some driving functions (e.g., steering, acceleration, brake, lane centering, adaptive cruise control, etc.) while the driver operates the vehicle in a normal operation section, and the driver is expected to determine an operation state and/or timing of the system, perform other driving functions, and cope with (e.g., resolve) emergency situations. At autonomous driving level 2, the SAE classification standard may correspond to "partial automation," in which the system performs steering, acceleration, and/or braking under the supervision of the driver, and the driver is expected to determine an operation state and/or timing of the system, perform other driving functions, and cope with (e.g., resolve) emergency situations. At autonomous driving level 3, the SAE classification standard may correspond to "conditional automation," in which the system drives the vehicle (e.g., performs driving functions such as steering, acceleration, and/or braking) under limited conditions but transfer driving control to the driver when the required conditions are not met, and the driver is expected to determine an operation state and/or timing of the system, and take over control in emergency situations but do not otherwise operate the vehicle (e.g., steer, accelerate, and/or brake). At autonomous driving level 4, the SAE classification standard may correspond to "high automation," in which the system performs all driving functions, and the driver is expected to take control of the vehicle only in emergency situations. At autonomous driving level 5, the SAE classification standard may correspond to "full automation," in which the system performs full driving functions without any aid from the driver including in emergency situations, and the driver is not expected to perform any driving functions other than determining the operating state of the system. Although the present disclosure may apply the SAE classification standard for autonomous driving classification, other classification methods and/or algorithms may be used in one or more configurations described herein. One or more features associated with autonomous driving control may be activated based on configured autonomous driving control setting(s) (e.g., based on at least one of: an autonomous driving classification, a selection of an autonomous driving level for a vehicle, etc.).

Based on one or more features (e.g., evaluating the engagement state of the driver) described herein, an operation of the vehicle may be controlled. The vehicle control may include various operational controls associated with the vehicle (e.g., autonomous driving control, sensor control, braking control, braking time control, acceleration control, acceleration change rate control, alarm timing control, forward collision warning time control, etc.).

One or more auxiliary devices (e.g., engine brake, exhaust brake, hydraulic retarder, electric retarder, regenerative brake, etc.) may also be controlled, for example, based on one or more features (e.g., evaluating the engagement state of the driver) described herein. One or more communication devices (e.g., a modem, a network adapter, a radio transceiver, an antenna, etc., that is capable of communicating via one or more wired or wireless communication protocols, such as Ethernet, Wi-Fi, near-field communication (NFC), Bluetooth, Long-Term Evolution (LTE), 5G New Radio (NR), vehicle-to-everything (V2X), etc.) may also be controlled, for example, based on one or more features (e.g., evaluating the engagement state of the driver) described herein.

Minimum risk maneuver (MRM) operation(s) may also be controlled, for example, based on one or more features (e.g., evaluating the engagement state of the driver) described herein. A minimal risk maneuvering operation (e.g., a minimal risk maneuver, a minimum risk maneuver) may be a maneuvering operation of a vehicle to minimize (e.g., reduce) a risk of collision with surrounding vehicles in order to reach a lowered (e.g., minimum) risk state. A minimal risk maneuver may be an operation that may be activated during autonomous driving of the vehicle when a driver is unable to respond to a request to intervene. During the minimal risk maneuver, one or more processors of the vehicle may control a driving operation of the vehicle for a set period of time.

Biased driving operation(s) may also be controlled, for example, based on one or more features (e.g., evaluating the engagement state of the driver) described herein. A driving control apparatus may perform a biased driving control. To perform a biased driving, the driving control apparatus may control the vehicle to drive in a lane by maintaining a lateral distance between the position of the center of the vehicle and the center of the lane. For example, the driving control apparatus may control the vehicle to stay in the lane but not in the center of the lane.

The driving control apparatus may identify a biased target lateral distance for biased driving control. For example, a biased target lateral distance may comprise an intentionally adjusted lateral distance that a vehicle may aim to maintain from a reference point, such as the center of a lane or another vehicle, during maneuvers such as lane changes. This adjustment may be made to improve the vehicle's stability, safety, and/or performance under varying driving conditions, etc. For example, during a lane change, the driving control system may bias the lateral distance to keep a safer gap from adjacent vehicles, considering factors such as the vehicle's speed, road conditions, and/or the presence of obstacles, etc.

One or more sensors (e.g., IMU sensors, camera, LIDAR, RADAR, blind spot monitoring sensor, line departure warning sensor, parking sensor, light sensor, rain sensor, traction control sensor, anti-lock braking system sensor, tire pressure monitoring sensor, seatbelt sensor, airbag sensor, fuel sensor, emission sensor, throttle position sensor, inverter, converter, motor controller, power distribution unit, high-voltage wiring and connectors, auxiliary power modules, charging interface, etc.) may also be controlled, for example, based on one or more features (e.g., evaluating the engagement state of the driver) described herein.

An operation control for autonomous driving (e.g., controlling an autonomous driving function) of the vehicle may include various driving control of the vehicle by the vehicle control device (e.g., acceleration, deceleration, steering control, gear shifting control, braking system control, traction control, stability control, cruise control, lane keeping assist control, collision avoidance system control, emergency brake assistance control, traffic sign recognition control, adaptive headlight control, driver warning control, autonomous driving operational design domain (ODD) control, etc.).

An autonomous driving level and/or autonomous driving activation/deactivation may also be controlled, for example, based on one or more features (e.g., evaluating the engagement state of the driver) described herein. A driving control apparatus may perform an autonomous driving level control (e.g., a change of an autonomous driving level, a change of a required user attentiveness, etc.) or cause deactivation of an autonomous driving operation. For example, by changing the required user attentiveness, the driver may be required to place his/her hands on the driving wheel more often (e.g., at least once in a threshold time period, such as five second, 30 seconds, 1 minute, etc.). By changing the required user attentiveness, the driver may be required to look ahead more often (e.g., at least once in a threshold time period, such as five second, 30 seconds, 1 minute, etc.). By changing the autonomous driving level, one or more video contents might not be displayed on a display of the vehicle.

In the various flowcharts of the present disclosure, one or more operations may be omitted or performed in any other order. At least some of the example examples of the present disclosure may be performed at any particular or arbitrary point in each operation of the flowchart. Various flowcharts of the present disclosure may be performed by at least one of an autonomous driving control device (also referred to as a driving automation device or driving automation system), a processor 130, a control unit 100, an autonomous vehicle 10, or a computer program.

Hereinafter, one or more example examples will be described in detail with reference to the accompanying drawings, but identical or corresponding components are denoted by the same reference numerals regardless of figure numbers, and redundant descriptions thereof will be omitted.

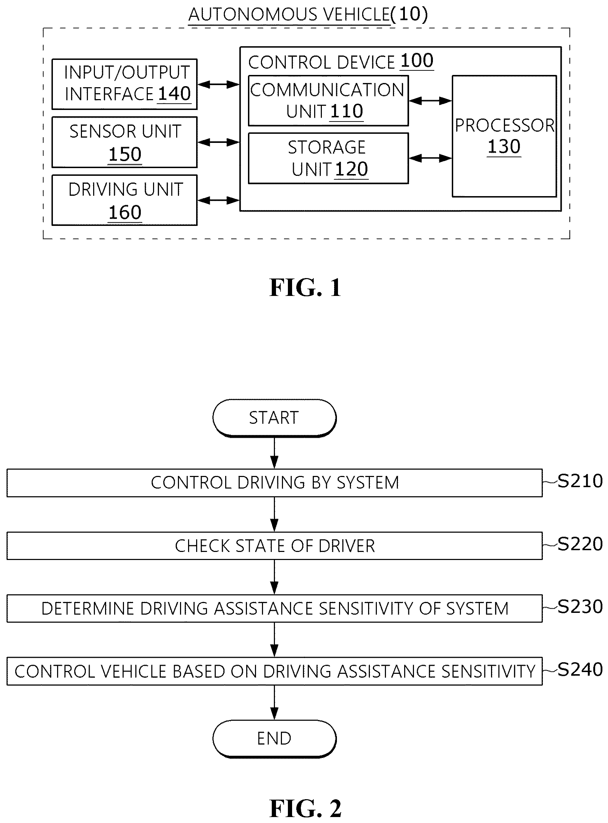

FIG. 1 is a block diagram of an autonomous vehicle 10 according to an example.

The autonomous vehicle 10 may include a control device 100, a communication unit 110, a storage unit 120, a processor 130, an input/output interface 140, a sensor unit 150, and/or a driving unit 160. Each of these components of FIG. 1 may be implemented inside the vehicle.

The autonomous vehicle 10 may be dynamically controlled in various driving situations through an autonomous driving system. For example, the autonomous driving system may provide both driving stability and convenience through longitudinal control (acceleration and deceleration) and lateral control (lane keeping and changing) of the autonomous vehicle 10. Such an autonomous driving system may monitor the driving environment through sensors and a control device, and process data in real time to adjust the position, driving speed, direction, and the like of the vehicle.

The control device 100 may be integrally formed with other internal components of the vehicle. Additionally and/or alternatively, the control device 100 may be implemented as a separate device and may communicate with the internal components of the vehicle via various connection means (e.g., a Controller Area Network (CAN) bus, a wireless network, a wired connection, etc.). The control device 100 includes the communication unit 110, the storage unit 120, and the processor 130 to manage the longitudinal and lateral control of the vehicle, and may additionally include other components such as the input/output interface 140, the sensor unit 150, and the driving unit 160 to perform complex control functions according to a driving situation.

The communication unit 110 may share data between systems by communicating with other control devices inside the vehicle, or may transmit and receive various information through a connection with an exterior of the vehicle. The communication unit 110 transmits control signals and data between internal components using various in-vehicle communication methods such as Controller Area Network (CAN) communication and Ethernet, and may link driving information and external data in real time through communication with a user terminal, other vehicles (V2V: Vehicle-to-Vehicle), infrastructure (V2I: Vehicle-to-Infrastructure), or an external server.

The communication unit 110 may perform short-range communication, Vehicle-to-Everything (V2X) communication, optical communication, broadcast transmission and reception, and Intelligent Transport Systems (ITS) communication functions, and may include a Global Navigation Satellite System (GNSS) module (e.g., a GPS module). For example, the communication unit 110 may support stable data transmission at a short range using various wireless communication technologies such as Bluetooth^{®}, Radio Frequency Identification (RFID), Infrared Data Association (IrDA), Ultra Wideband (UWB), ZigBee, Near Field Communication (NFC), Wi-Fi, Wi-Fi Direct, and Wireless USB. Furthermore, the communication unit 110 may perform long-range communication via a mobile communication module (e.g., based on Long-Term Evolution (LTE) and 5G networks) and a wireless Internet module, and may improve the performance of the autonomous driving system by receiving real-time data or interworking with a cloud.

The storage unit 120 may include various types of memory capable of storing data, and may be integrated into the control device 100 or the processor 130, or may be configured in the form of a separate module. The storage unit 120 may include non-volatile memory and volatile memory, and may implement a memory system of various capacities and performances by combining them.

According to an example, the storage unit 120 may be configured to store driver state information (such as gaze direction and hand position) and corresponding driving assistance sensitivity settings, which can be referenced by the system when analyzing the driver's current state.

Furthermore, the storage unit 120 may store data such as a sensitivity setting table corresponding to whether hands-off is permitted, inter-vehicle distance levels, timing for collision avoidance warnings/control, and lane keeping settings, thereby enabling the processor to perform vehicle control based on this data.

According to an example, the storage unit 120 may store an autonomous driving system.

The autonomous driving system may be configured to analyze driver state information (such as gaze direction and hand position) and the vehicle's driving environment to determine the driving assistance sensitivity for parameters like inter-vehicle distance, collision avoidance warning/control timing, and/or lane keeping settings, and to control the vehicle accordingly.

Moreover, the storage unit 120 may also store functions of a general autonomous driving system, thereby improving the vehicle's autonomous driving performance.

For example, the storage unit 120 may store data and algorithms related to autonomous driving functions such as path planning, obstacle avoidance, lane changing, speed control, traffic signal recognition, and/or stop-and-go. This can also include functions such as adjusting sensitivity based on whether hands-off is permitted, switching driving modes using vehicle sensor data, and optimizing the driving path.

The storage unit 120 may store instructions that can execute the various examples of the present invention. These instructions may include instructions configured to: check the driver's state via the vehicle's sensor unit during driving control by the system; determine the system's driving assistance sensitivity based on the identified driver state; and/or control the vehicle in accordance with the determined driving assistance sensitivity.

Additionally, the storage unit 120 may store data that the processor 130 can utilize based on pre-stored values corresponding to the driver's state, in order to control at least one of the autonomous vehicle's driving behavior or warning output.

In this case, the pre-stored value corresponding to the driver's state is a setting value that corresponds to the driving assistance sensitivity, and may include, for example, control criteria related to inter-vehicle distance levels, collision avoidance warning timing, steering intervention strength, and/or the intensity and frequency of warning outputs.

The processor 130 may communicate with various internal components of the autonomous vehicle 10, such as the communication unit 110, the storage unit 120, the input/output interface 140, the sensor unit 150, and/or the driving unit 160, through an electrical or operative connection, and may control the operation of each component and perform data processing. The processor 130, as a central processing unit for executing instructions and calculating data, may perform vehicle control by collecting, processing, and analyzing data in real time according to the driving environment of the vehicle.

The processor 130 may be implemented in the form of hardware, software, or a combination thereof, and may, for example, perform vehicle control logic in the form of a microcontroller, an FPGA, or an ASIC. The processor 130 may additionally and/or alternatively include a multi-processor configuration for controlling complex autonomous driving and driver assistance systems. Such a processor configuration may support comprehensive control and stable performance of the vehicle through functions such as execution of autonomous driving and assistance systems, sensor data processing, communication data management, and driving-related determination functions.

According to an example of the present invention, the processor 130 is configured to determine a driver's state when a hands-off state of the driver is detected through a sensor unit 150 of an autonomous vehicle 10, and control at least one of a driving operation or a warning output of the autonomous vehicle 10 based on the determined driver's state.

The input/output interface 140 serves to receive an input related to vehicle control from a user and transmit vehicle control status and system operation information to the user.

The input/output interface 140 may perform a function of receiving various inputs from a user and transmitting a vehicle control status to the user. For example, the input/output interface 140 may include at least one of: an input through a physical button provided inside the autonomous vehicle 10, an input through a soft button displayed on a display of the autonomous vehicle 10, a user's voice input, and/or a user's gesture input.

The input module may include a physical button, a soft key on a touch display, a voice recognition function, a gesture recognition function, and/or the like, through which a user may input commands such as an autonomous driving system activation request, a function switch, and/or a driving assistance setting.

**In** addition, the input module may receive a user input through an input device provided on a steering wheel of the autonomous vehicle 10. For example, through a button mounted on the steering wheel, a driver may input a command to activate or deactivate the autonomous driving system even during driving. Such a steering wheel-based input may support a user to promptly control the autonomous driving function by providing an intuitive interface.

The output module includes a display, an audio module (e.g., a speaker), a haptic module, and the like, and may provide, to a user, a status of the autonomous driving system, a control switch request notification, and information on whether a function is activated, in visual, auditory, and tactile forms.

In addition, a user may intuitively check the status of the autonomous driving system through a head-up display (HUD), a cluster, an Audio Video Navigation (AVN) system, and a display embedded in a rear-view mirror or a side mirror. Besides, the status of the autonomous driving system may be checked through a display of a console installed in each row of the autonomous vehicle 10, an application of a user terminal, and the like.

According to an example of the present invention, the input/output interface 140 may output a control state associated with at least one of a driving operation or a warning output of the autonomous vehicle 10. For example, state information such as the set level for the vehicle's inter-vehicle distance, the activation status of the collision avoidance function, the intervention strength of the lane keeping assist function, or the current intensity and frequency of warning outputs may be provided to the driver in real-time via a display, a head-up display (HUD), an audio module, or a haptic module.

The sensor unit 150 may include a plurality of sensors that detect various driving and environmental information in real time to support stable operation of the autonomous driving system. The sensor unit 150 may measure a distance to and a speed of a surrounding object through long-range detection sensors such as a Radio Detection and Ranging (RADAR) sensor and a Light Detection and Ranging (LIDAR) sensor, and/or may include an ultrasonic sensor to detect an object near the autonomous vehicle 10.

In addition, the sensor unit 150 may include a position sensor, such as a GPS, to collect position information of the autonomous vehicle 10, and an optical sensor to provide surrounding illumination information, thereby allowing the system to respond to changes in light. Furthermore, the sensor unit 150 may monitor a driving control state and a user's action of the autonomous vehicle 10 by including sensors such as an acceleration pedal and deceleration pedal sensor, a steering wheel detection sensor, and a seating sensor.

The sensor unit 150 may include a camera. The camera may be classified into an external camera and an internal camera. The external camera may be configured to recognize the road and the surrounding environment, and the internal camera may comprehensively grasp the situation inside and outside the vehicle by detecting a driver's state (e.g., eye tracking, drowsiness detection) or an indoor situation.

The sensor unit 150 may collect a driver's biometric information or various environmental data by including a heart rate sensor, a pressure sensor, an infrared sensor, and the like.

According to an example, a camera may detect the user's gaze direction. For example, the camera may determine the direction of gaze based on the user's face orientation, head angle, and pupil movements. To this end, the camera may track the user's pupils in real-time to detect whether the user is gazing at a specific area (e.g., the steering wheel, the road ahead, the instrument cluster, etc.).

Additionally, the sensor unit 150 may also detect the gaze direction by analyzing corneal reflection data from the pupils using infrared technology. Through this, the driver's gaze direction may be detected even in dark environments.

According to an example, the sensor unit 150 may detect input from the user's body on the steering wheel.

For example, the sensor unit 150 may determine the driver's Hands-Off state by detecting whether the user's hand is holding the steering wheel, a contact area, a contact pressure, changes in hand position, and the like.

The sensor unit 150 may detect input from the user's body on the steering wheel and may provide the detected data to the processor 130.

The sensor unit 150 or the processor 130 may detect whether the driver is holding the steering wheel, whether the driver has released the steering wheel, whether the hands are still within the driver's seat range when the steering wheel is released, whether the hands have moved outside the driver's seat range after releasing the steering wheel, whether the driver is holding another object (e.g., a mobile phone, a drink, etc.) with one hand, or whether the driver is holding another object with both hands.

To this end, the sensor unit 150 may include a capacitive touch sensor, a pressure sensor, a torque sensor, or a camera-based sensor, and these sensors may be configured to be included in the steering wheel or located in an area adjacent to the steering wheel.

Data detected by the sensor unit 150 is transmitted to the processor 130, and the processor 130 may analyze the data to determine the driver's state and adjust the driving assistance sensitivity accordingly.

The processor 130 may comprehensively analyze the sensor data to determine whether the driver is holding or has released the steering wheel.

For example, if the driver has released their hands, the processor 130 may additionally analyze the position of the hands to determine if they are within or outside the driver's seat area.

For example, if the driver is holding a specific object, the processor 130 may combine camera input and sensor data to recognize this and may adjust the vehicle control strategy.

Furthermore, the processor 130 may utilize a deep learning-based neural network model to learn from the data detected by the sensor unit 150 and may predict the driver's hand movement patterns.

For example, if the driver momentarily takes their hands off the steering wheel and then grips it again, this may be recognized as a simple transient action, and the system may re-determine the driver's state.

On the other hand, if the driver continuously keeps their hands off the wheel or is holding another object, the processor 130 may enhance the driving assistance intervention by changing the driving assistance sensitivity.

According to an example, the processor 130 may determine the driver's state by combining a sensor-based detection method and a deep learning-based method.

For example, as a sensor-based detection method, the processor 130 may detect hand contact via a capacitive touch sensor, may measure contact strength using a pressure sensor, and may detect hand position and movement using an infrared sensor or a camera.

As a deep learning-based method, the processor 130 may learn hand movement patterns through video analysis using a camera and may utilize a machine learning model to recognize whether the driver's hands are holding a specific object.

The processor 130 may determine the driver's state using at least one of the sensor-based detection method or the deep learning-based method described above.

The driving unit 160 may include various components that provide a driving force necessary for driving of the autonomous vehicle 10 and control an operation of the vehicle according to a command output from the control device 100. The driving unit 160 may be composed of devices that generate and transmit power of the vehicle, such as an engine, a motor, a transmission, and/or a wheel drive system, and/or a controller that controls them, and/or through these components, acceleration, deceleration, and/or a direction change of the autonomous vehicle 10 may be performed.

The driving unit 160 may be controlled to perform longitudinal control (acceleration and deceleration) and lateral control (lane keeping and changing) to maintain driving stability. For example, upon receiving a command from the control device 100, the driving unit 160 may allow the vehicle to follow a driving path by adjusting an output of a motor or a rotation speed and direction of wheels.

In addition, the driving unit 160 may include a brake system to reduce the vehicle speed or stop the vehicle during driving. The driving unit 160 may control the autonomous vehicle 10 through an electric motor in the case of an electric vehicle, and based on engine output in the case of an internal combustion engine vehicle.

FIG. 2 is a flowchart of an operation of controlling a vehicle according to an example.

The control device 100 may control the driving of the vehicle 10 by the autonomous driving system (S210).

The control device 100 may perform a lane keeping function of the vehicle based on lane information of a road, and may adjust steering so that the vehicle does not deviate from the lane. The lane keeping function may include a lane departure warning and a lane keeping assist function, and may support lane keeping through steering intervention when necessary.

In addition, the control device 100 may perform control to maintain a vehicle interval by detecting an obstacle in front of the vehicle or a distance from a preceding vehicle. To this end, the control device 100 may measure the speed and distance of the preceding vehicle using sensors such as a radar sensor, a LIDAR, and a camera, and perform an adaptive cruise control function.

In particular, when the autonomous driving system controls the vehicle without the intervention of the driver, the control device 100 may adjust the speed in consideration of the traffic flow, and perform control such as lane change or stop as necessary.

Next, the control device 100 may check the state of the driver (S220).

The control device 100 may collect data related to the driver state from the sensor unit 150, and analyze the data to determine the state of the driver, that is, whether the driver is involved in driving.

In the driver state checking process, the control device 100 may sense a gaze direction of the driver, a hand position, whether or not the steering wheel is in contact, a body movement, and the like. For this purpose, various sensing devices such as a capacitive touch sensor, a pressure sensor, a torque sensor, an infrared sensor, a camera, and an eye tracking sensor may be utilized.

For example, the control device 100 may detect whether the driver is holding the steering wheel and determine whether the driver is in a hands-off state. In addition, it is possible to evaluate the attention concentration of the driver by analyzing whether the driver's gaze is looking forward or moving to the side or the lower end (e.g., a mobile phone).

Additionally, the control device 100 may compare and analyze the data sensed by the sensor with a reference value stored in the storage unit 120 to compare with a normal operation pattern of the driver. In this process, a deep learning-based artificial intelligence (AI) (e.g., machine learning) model may be used to learn a behavior pattern of the driver, and abnormal behavior (e.g., drowsy driving, a case where a hand is removed from a steering wheel and operation is not performed for a long time, etc.) may be detected.

Next, the control device 100 may determine the driving assistance sensitivity of the system (S230).

The control device 100 may analyze the driver state information, and determine the driving assistance sensitivity appropriate for each situation by referring to the sensitivity adjustment table for each driver state and whether to allow the hands-off stored in the storage unit 120.

In addition, the control device 100 may learn the driving habits of individual drivers through machine learning (e.g., deep learning)-based driving pattern analysis, and perform personalized sensitivity adjustment.

Next, the control device 100 may control the vehicle by reflecting the driving assistance sensitivity (S240).

The control device 100 may apply the driving assistance sensitivity to the warning system, acceleration, deceleration, and steering of the vehicle in the longitudinal direction or the lateral direction to control the driving of the vehicle in real time.

The control device 100 may calculate a parameter necessary for the driving assistance according to the determined driving assistance sensitivity, and may control the driving of the vehicle or control the warning system according to the calculated value.

When the driving assistance sensitivity is increased (e.g., when the driver is in a careless state), the control device 100 may increase the inter-vehicle distance more than the preset inter-vehicle distance and limit the acceleration to ensure safety. In contrast, when the driving assistance sensitivity is lowered (e.g., when the driver actively intervenes in driving), the control device 100 may reduce the inter-vehicle distance.

When the driving assistance sensitivity is increased, the control device 100 may early activate the collision prevention warning time point and perform automatic deceleration or emergency braking as necessary.

When the driving assistance sensitivity is increased, the control device 100 may increase the intervention strength of the lane keeping assistance function by the system to assist the vehicle so as not to deviate from the lane.

The control device 100 may adjust the warning intensity depending on the driving assistance sensitivity, and may provide feedback to the driver in a manner such as visual (display, HUD), auditory (warning sound), and tactile (steering wheel vibration).

In addition, when the driver continues to be in a dangerous state, the control device 100 may increase the warning frequency or perform emergency intervention.

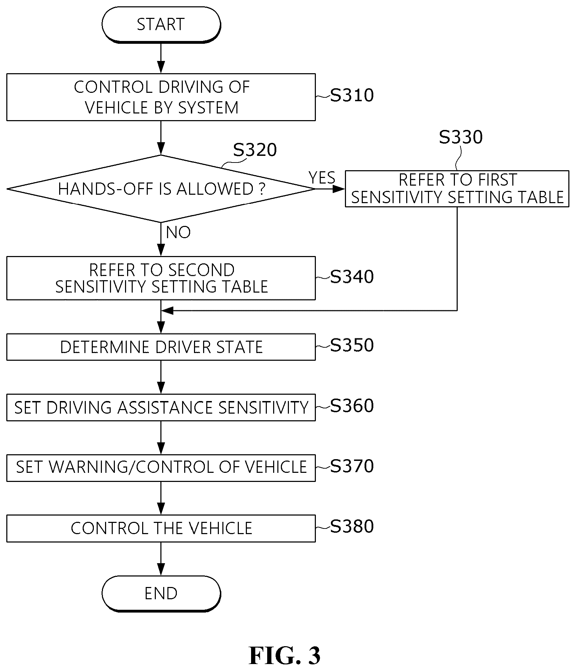

FIG. 3 is a specific flowchart of an operation of controlling a vehicle according to an example.

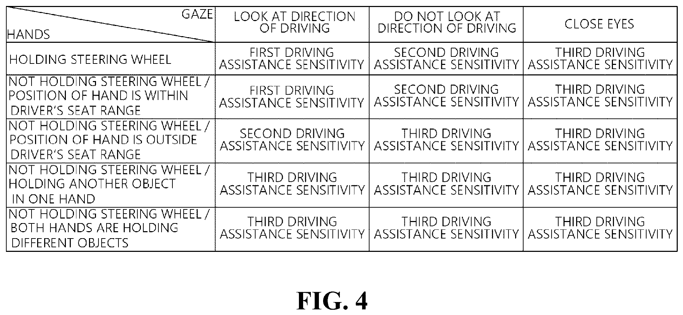

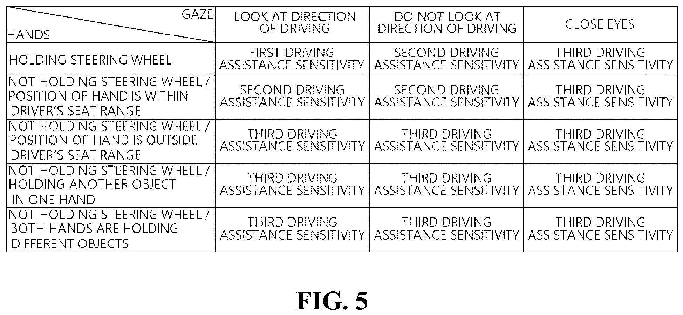

The content of FIG. 3 that overlaps with the content of FIG. 2 is omitted. For a description of FIG. 3, refer to FIGS. 4 to 5. FIG. 4 is a sensitivity setting table in a section where hands-free is allowed by the system, and FIG. 5 is a sensitivity setting table in a section where the hands-free is not allowed by the system.

The control device 100 may control the driving of the vehicle 10 by the autonomous driving system (S310).

The control device 100 may control the vehicle by analyzing the position, the speed, the lane information, the distance from a preceding vehicle, and the surrounding traffic flow of the vehicle. In addition, it is possible to adjust the degree of intervention of the driving assistance system in consideration of the driving mode of the vehicle (e.g., highway driving, city driving, congestion section, etc.).

Next, the control device 100 may check whether the driver is currently in a hands-free state in which hands-off of the driver is allowed (S320).

To this end, the control device 100 may refer to the hands-free allowable condition of the vehicle stored in the storage unit 120, and analyze the current state and the driving environment of the vehicle through the sensor unit 150.

According to an example, the control device 100 may apply the driving assistance sensitivity differently depending on whether the hands-off state of the driver in the system is an acceptable state and a non-acceptable state, respectively.

For example, if the hands-off is allowed by the system (S320: Yes), the control device 100 may refer to a first sensitivity setting table (S330) and determine the driving assistance sensitivity (S360) according to the driver state (S350).

If the hands-off is not allowed by the system (S320: NO), the control device 100 may refer to a second sensitivity setting table (S340) and determine the driving assistance sensitivity (S360) according to the driver state (S350).

The first sensitivity setting table is shown in FIG. 4. This first sensitivity setting table corresponds to data that the processor 130 refers to when hands-off is allowed.

The driver state may be implemented in a configuration that includes at least one of a contact area of a user's body with respect to a steering wheel of the autonomous vehicle, a position of the user's body, and/or an object adjacent to the user's body. For example, the state of the driver may be determined based on whether the driver is holding the steering wheel, whether the contact area of the hand is narrowed or the position of the hand is away from the steering wheel, and/or whether the user's hand is holding another object such as a mobile phone or a beverage. This information on the driver state may be used as a vertical axis item in the sensitivity setting table, and utilized to determine the driving assistance sensitivity corresponding to each situation.

The control device 100 may apply the driving assistance sensitivity differently according to at least one of a contact area of the user body with the steering wheel of the vehicle 10, a position of the user body, and/or an object adjacent to the user body.

In the first sensitivity setting table of FIG. 4, the vertical axis may be set with items related to the user's body (e.g., hands) for the steering wheel.

For example, it may be set whether the driver is holding the steering wheel, whether the driver is not holding the steering wheel and the position of the hand is within the driver's seat range, whether the driver is not holding the steering wheel and a position of a hand is outside the driver's seat range, whether the driver is not holding the steering wheel, and holding another object in one hand, and/or whether the driver is not holding the steering wheel and both hands are holding different objects.

The driver state may include a gaze direction of the driver. Further, the driver state may include at least one of an eye closing ratio, an eye closing duration, and/or a combination thereof of the driver. The control device 100 may differently apply the driving assistance sensitivity based on at least one of a gaze shape of the driver, that is, a gaze direction, an eye closing ratio, an eye closing duration, or a combination thereof.

In the first sensitivity setting table of FIG. 4, items related to a gaze shape of the driver may be set for the horizontal axis.

For example, the gaze shape may be divided into items of whether the eyes are looking at the driving direction, not looking in the driving direction, and whether the eyes are being closed.

In the first sensitivity setting table, three levels of driving assistance sensitivity may be set according to the horizontal axis item and the vertical axis item. For example, the first driving assistance sensitivity, the second driving assistance sensitivity, and the third driving assistance sensitivity may be set.

According to an example of the present invention, the control device 100 may control the autonomous vehicle 10 to increase the inter-vehicle distance of the autonomous vehicle 10 more than the preset inter-vehicle distance when the gaze of the driver is out of the driving direction for more than the preset time or when the eye-closed state is confirmed.

For example, when the driver is holding the steering wheel and is looking at the driving direction, the driver is classified as the first driving assistance sensitivity having the lowest sensitivity.

The first driving assistance sensitivity may be set, for example, to keep the inter-vehicle distance in a manual setting, to keep the anti-collision warning/control in a manual setting and to keep the lane keeping function in a manual setting.

In addition, when the driver is holding the steering wheel and does not look in the driving direction, it may be classified as a second driving assistance sensitivity that is higher than the first driving assistance sensitivity.

The second driving assistance sensitivity may be set, for example, to keep the inter-vehicle distance intermediate (e.g., two to three steps), to keep the anti-collision warning/control to a normal setting, and to keep the lane keeping function (e.g, warning, assistance control) to a normal setting.

In addition, when the driver is holding the steering wheel but closing his/her eyes, it may be classified as a third driving assistance sensitivity that is higher than the second driving assistance sensitivity.

The third driving assistance sensitivity may be set, for example, to keep the inter-vehicle distance the longest (e.g., four steps), to keep the anti-collision warning/control as normal or fast, to output the lane keeping warning earlier, or to strongly intervene in the assistance control for lane keeping.

Contents set in the first driving assistance sensitivity, the second driving assistance sensitivity, and the third driving assistance sensitivity are exemplary, and may be set in various manners and steps.

The second sensitivity setting table is shown in FIG. 5. This second sensitivity setting table corresponds to data that the processor 130 refers to when hands-free is not allowed by the system. That is, when the system does not allow the hands-off state of the driver, the processor 130 of the control device 100 may apply a more conservative criterion with reference to the second sensitivity setting table rather than the first sensitivity setting table.

In the second sensitivity setting table, the driving assistance sensitivity is classified more conservatively than in the first sensitivity setting table. In particular, the setting for the hand position may be applied differently because the hands-free state is not allowed.

For example, in the first sensitivity setting table of FIG. 4, when the driver is not holding the steering wheel, has the hand position within the driver's seat range, and is looking at the driving direction, it is classified as the first driving assistance sensitivity, but in the second sensitivity setting table of FIGS. 5, it may be classified as the second driving assistance sensitivity.

According to the foregoing example, by distinguishing the first sensitivity setting table from the second sensitivity setting table, the driving assistance sensitivity may be differentially adjusted in states where hands-free driving is possible and where it is not possible. In this way, a safer and more flexible driving assistance function may be operated depending on the state of the driver.

According to an example, the control device 100 may output the determined driving assistance sensitivity through the input/output interface 140 of the vehicle 10 in a visual, audible, or tactile manner.

Referring again to FIG. 3, the control device 100 may calculate a parameter for a warning system or driving control of the vehicle by reflecting the determined driving assistance sensitivity (S370).

According to an example, the control device 100 may calculate an acceleration and deceleration control amount for longitudinal control of the vehicle based on the determined driving assistance sensitivity.

The control device 100 may calculate control parameters for maintaining the inter-vehicle distance and adjusting the deceleration in consideration of the vehicle speed, the distance from the preceding vehicle, the driving environment, and the driver state.

For example, the control device 100 may calculate a target headway distance for setting the inter-vehicle distance maintenance, and determine a deceleration curve and an acceleration gradient.

In addition, when the driver is in the hands-off state or the gaze deviates from the front, the parameter may be changed in such a manner that the deceleration response is adjusted more quickly and the inter-vehicle distance is increased.

According to an example, the control device 100 may also calculate a longitudinal control amount for collision prevention.

The control device 100 may calculate the Time-to-Collision (TTC) by utilizing the state of the driver and the distance information from the preceding vehicle, and adjust the autonomous deceleration rate when necessary.

For example, the control device 100 may calculate a threshold value for operating the emergency braking system when the risk of collision is high.

According to an example, the control device 100 may calculate a lateral control amount to perform lane keeping or lane changing of the vehicle.

The control device 100 may determine a steering angle and a steering torque so that the vehicle 10 may maintain the center of the lane, and may adjust the steering assistance intervention strength depending on the driving assistance sensitivity.

For example, when the driver is holding the steering wheel and is looking at the driving direction, the control device 100 may minimize the steering intervention, and conversely, when the driver is in the hands-off state or is out of gaze from the front, the control device 100 may increase the steering torque intervention intensity and adjust the vehicle to maintain the center of the lane.

In addition, the control device 100 may calculate the steering angle velocity and the steering ratio to perform the lane change assistance, and when the sensitivity is high, the steering assistance intervention may be more actively performed when the lane is changed.

According to an example, the control device 100 may calculate a parameter for adjusting the intensity and frequency for the visual, auditory and tactile warning system in consideration of the state of the driver and the level of danger. The control device 100 may control a warning output including at least one of an intensity of the warning, a duration of the warning, or a repetition period of the warning.

When the driver is in the hands-off state or deviates the gaze from the front, the control device 100 may adjust the parameters to increase the strength of the warning system or increase the warning frequency.

For example, the control device 100 may determine the intensity, the number of repetitions, and the frequency of the warning sound, and may activate an additional warning manner such as steering wheel vibration (tactile warning) or dashboard/head-up display (HUD) display (visual warning) when necessary.

In addition, the control device 100 may calculate a threshold value at which the collision prevention warning system is to be operated, and may set the early warning to be activated as the degree of the driver's driving intervention is lower.

According to an example, the driving assistance sensitivity may be set as a parameter affecting the longitudinal and lateral control amounts of the vehicle as well as the warning system.

For example, the control device 100 may calculate parameters such as a coefficient for adjusting acceleration and deceleration responses, an inter-vehicle distance adjustment coefficient, a steering intervention intensity coefficient, and a warning frequency adjustment coefficient with reference to the driving assistance sensitivity.

Next, the control device 100 may control the vehicle 10 using the parameter calculated in step S370 (S380).

According to various examples, the control of the vehicle 10 by the control device 100 may include control of an inter-vehicle distance, warning or control for preventing a collision with a preceding vehicle, warning or control to maintain a lane.

According to the above example, by automatically adjusting the driving assistance sensitivity based on the state of the driver, the system may actively intervene and improve safety even when the driver is inadvertent.

Although the preferable examples of the present disclosure have been described above, it will be understood by those of ordinary skill in the art that various modifications and changes may be made to the present disclosure without departing from the spirit and scope of the disclosure as defined in the following claims.

## Claims

1. An autonomous vehicle control device, comprising:
a memory; and
a processor connected to the memory, wherein the processor is configured to cause an autonomous vehicle to:
determine, based on detecting a hands-off state of a driver of the autonomous vehicle via a sensor unit of the autonomous vehicle, a driver state, and
control, based on the driver state, at least one of:
a driving operation of the autonomous vehicle, or
a warning output of the autonomous vehicle.

2. The autonomous vehicle control device of claim 1, wherein the processor is configured to cause the autonomous vehicle to control the at least one of the driving operation or the warning output based on whether the hands-off state is permitted.

3. The autonomous vehicle control device of claim 1 or 2, wherein the driver state comprises at least one of:
a contact area between a body of the driver and a steering wheel of the autonomous vehicle,
a position of the body of the driver, or
an object adjacent to the body of the driver.

4. The autonomous vehicle control device of any one of claims 1 to 3, wherein the driver state comprises a gaze direction of the driver, and/or
at least one of:
an eye closing ratio of the driver, or
an eye closing duration of the driver.

5. The autonomous vehicle control device of any one of claims 1 to 4, wherein the driving operation of the autonomous vehicle comprises inter-vehicle distance control of the autonomous vehicle, and/or control of at least one of a steering angle or a steering torque of the autonomous vehicle.

6. The autonomous vehicle control device of any one of claims 1 to 5, wherein the processor is configured to cause the autonomous vehicle to:
adjust an inter-vehicle distance of the autonomous vehicle to be increased more than a preset inter-vehicle distance based on one or more of:
determining that a gaze of the driver is out of a driving direction for more than a preset time, or
confirming an eye-closed state of the driver.

7. The autonomous vehicle control device of any one of claims 1 to 6, wherein the control of the warning output comprises controlling at least one of:
an intensity of the warning output,
a duration of the warning output, or
a repetition period of the warning output.

8. The autonomous vehicle control device of any one of claims 1 to 7, wherein the processor is further configured to cause the autonomous vehicle to:
output, via a display, a control state associated with at least one of:
the driving operation of the autonomous vehicle, or
the warning output of the autonomous vehicle.

9. A method performed by an apparatus of an autonomous vehicle, the method comprising:
determining, based on detecting a hands-off state of a driver of the autonomous vehicle via a sensor unit of the autonomous vehicle, a driver state; and
controlling, based on the driver state, at least one of:
a driving operation of the autonomous vehicle, or
a warning output of the autonomous vehicle.

10. The method of claim 9, wherein the controlling the at least one of the driving operation or the warning output is based on whether the hands-off state is permitted.

11. The method of claim 9 or 10, wherein the driver state comprises at least one of:
a contact area between a body of the driver and a steering wheel of the autonomous vehicle,
a position of the body of the driver, or
an object adjacent to the body of the driver.

12. The method of any one of claims 9 to 11, wherein the driver state comprises a gaze direction of the driver, and/or
at least one of:
an eye closing ratio of the driver, or
an eye closing duration of the driver.

13. The method of any one of claims 9 to 12, wherein the driving operation of the autonomous vehicle comprises inter-vehicle distance control of the autonomous vehicle, and/or
control of at least one of:
a steering angle of the autonomous vehicle, or
a steering torque of the autonomous vehicle.

14. The method of any one of claims 9 to 13, wherein the controlling the driving operation comprises:
causing an inter-vehicle distance of the autonomous vehicle to be increased more than a preset inter-vehicle distance based on one or more of:
determining that a gaze of the driver is out of a driving direction for more than a preset time, or
confirming an eye-closed state of the driver, and/or
wherein the controlling the warning output comprises controlling at least one of:
an intensity of the warning output,
a duration of the warning output, or
a repetition period of the warning output.

15. A computing device for monitoring a driver during autonomous operation of a vehicle, the computing device comprising:
one or more processors; and
memory storing instructions that, when executed by the one or more processors, cause the computing device to:
generate a behavior pattern of a driver of the vehicle by collecting, via one or more sensors of the vehicle, first sensor data corresponding to a body of the driver;
receive, from the one or more sensors of the vehicle and during autonomous driving operations of the vehicle, second sensor data corresponding to the body of the driver;
identifying a degree of driver attentiveness by comparing the second sensor data to the behavior pattern of the driver; and
cause, based on determining that the degree of driver attentiveness satisfies a threshold, the vehicle to operate in a safe driving mode by increasing an inter-vehicle distance of the vehicle to a value greater than a preset inter-vehicle distance.
